(19) Europäisches Patentamt
European Patent Office
Office européen des brevets

(11) **EP 4 413 973 A1**

(12) **EUROPEAN PATENT APPLICATION**
published in accordance with Art. 153(4) EPC

(43) Date of publication:
**14.08.2024 Bulletin 2024/33**

(21) Application number: **22878408.8**

(22) Date of filing: **29.09.2022**

(51) International Patent Classification (IPC):
*A61K 8/46* $^{(2006.01)}$     *A61Q 5/02* $^{(2006.01)}$
*A61Q 5/12* $^{(2006.01)}$     *A61Q 19/00* $^{(2006.01)}$
*A61Q 19/10* $^{(2006.01)}$

(52) Cooperative Patent Classification (CPC):
**A61K 8/46; A61Q 5/02; A61Q 5/12; A61Q 19/00; A61Q 19/10**

(86) International application number:
**PCT/JP2022/036341**

(87) International publication number:
**WO 2023/058538 (13.04.2023 Gazette 2023/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.10.2021 JP 2021163600**

(71) Applicant: **Kao Corporation**
**Tokyo 103-8210 (JP)**

(72) Inventor: **MARUYAMA, Aguri**
**Tokyo 131-8501 (JP)**

(74) Representative: **Hoffmann Eitle**
**Patent- und Rechtsanwälte PartmbB**
**Arabellastraße 30**
**81925 München (DE)**

(54) **METHOD FOR TREATING KERATINOUS MATERIAL**

(57) A method for treating a keratinous material, including the following Step (I) and Step (II) in this order: Step (I): a step of applying a cleansing agent composition (A) that contains an internal olefin sulfonic acid or a salt thereof (a) obtained by sulfonating a starting olefin having an average double bond position of position 3.9 or higher and position 4.5 or lower to a keratinous material; Step (II): a step of applying a conditioning agent composition (B) containing a cationic surfactant (b) to the keratinous material.

**Description**

Field of the Invention

The present invention relates to a method for treating a keratinous material.

Background of the Invention

As an anionic surfactant, internal olefin sulfonic acids having different numbers of carbon atoms are known. An internal olefin sulfonic acid salt can provide an excellent effect as an anionic surfactant, and thus, is used in a cleansing agent composition for a keratinous material, such as skin or hair.

For example, JP 2015-27975 A (PTL 1) discloses that a skin- or hair-cleansing agent composition that contains an internal olefin sulfonic acid salt having 12 or more and 24 or less carbon atoms and a cationic polymer or an amphoteric polymer can impart good foam durability, rinseability, finger combability in rinsing hair, hair softness after rinsing and towel drying, and moist feeling of skin.

JP 2015-178467 A (PTL 2) discloses that a composition that contains a sulfonic acid salt mixture containing an internal olefin sulfonic acid salt having 16 carbon atoms and an internal olefin sulfonic acid salt having 18 carbon atoms in a specific mass ratio and at least one foaming promoter or foaming booster has good foaming performance and good skin feeling after use, and is particularly useful as a cleansing product.

WO 2017/098637 (PTL 3) discloses that a surfactant composition that contains a specific amount of an internal olefin sulfonic acid salt having 16 or more and 18 or less carbon atoms, a specific amount of an $\alpha$-olefin sulfonic acid having 12 or more and 14 or less carbon atoms, and water can secure good preservation and fluidity while containing, at a high concentration, an internal olefin sulfonic acid salt having 16 or more and 18 or less carbon atoms which can exhibit superior foamability and cleansing ability.

Summary of the Invention

The present invention relates to the followings.

[1] A method for treating a keratinous material, including the following Step (I) and Step (II) in this order:

Step (I): a step of applying a cleansing agent composition (A) that contains an internal olefin sulfonic acid or a salt thereof (a) obtained by sulfonating a starting olefin having an average double bond position of position 3.9 or higher and position 4.5 or lower to a keratinous material;
Step (II): a step of applying a conditioning agent composition (B) containing a cationic surfactant (b) to the keratinous material.

[2] A cosmetic kit for treating a keratinous material, including:

a cleansing agent composition (A) containing an internal olefin sulfonic acid or a salt thereof (a) obtained by sulfonating a starting olefin having an average double bond position of position 3.9 or higher and position 4.5 or lower; and
a conditioning agent composition (B) containing a cationic surfactant (b).

[3] A composition for treating a keratinous material, being to be applied to a keratinous material before application of a conditioning agent composition (B) containing a cationic surfactant (b), the composition containing an internal olefin sulfonic acid or a salt thereof (a) obtained by sulfonating a starting olefin having an average double bond position of position 3.9 or higher and position 4.5 or lower.

Detailed Description of the Invention

PTL 1 discloses that a skin- or hair-cleansing agent composition having a cationic polymer or an amphoteric polymer blended therein can impart a good feeling to skin or hair while having a cleansing ability which is a basic performance. However, there has been a room for improvement in imparting a sufficient conditioning effect to a keratinous material, such as skin or hair, and further retaining the conditioning effect, with only use of the cleansing agent composition.

For example, in hair, by imparting a conditioning effect to a cleansed hair, effects of enhancing the lubricity and softness in rinsing hair, the hard-to-entangle property of hair in rinsing, and the setting property of dried hair are provided. In addition, from the viewpoint of reducing the time for the hair care action, an effect of enhancing the rate of drying of cleansed hair is also desired. However, even if a conditioning treatment is applied for imparting the above effects to the hair cleansed with a conventional cleansing agent composition, there is a problem in that the conditioning effect is lost when the hair is subsequently cleansed.

The present invention has an object to provide a method for treating a keratinous material, such as skin or hair, the method being capable of imparting a sufficient conditioning effect to a treated keratinous material, the method enhancing, particularly in the cleansed hair, the lubricity and softness in rinsing, the hard-to-entangle property in rinsing, the rate of drying, and the setting property of dried hair, and further being superior in the durability of the conditioning effect.

The present inventor has found that the above problem can be solved by a method for treating a keratinous material, the method including a step of applying a cleansing agent composition containing a specific internal olefin sulfonic acid or a salt thereof to a keratinous material and a step of applying a conditioning agent composition containing a cationic surfactant to the keratinous material in this order.

According to the present invention, it is possible to provide a method for treating a keratinous material, the method imparting a sufficient conditioning effect to a treated keratinous material, enhancing, particularly in the cleansed hair, the lubricity and softness in rinsing, the hard-to-entangle property in rinsing, the rate of drying, and the setting property of dried hair, and further being superior in the durability of the conditioning effect.

<Definition>

The phrase "containing a component X" in the present invention has the same meaning as "having a component X blended therein".

As the "keratinous material" in the present invention, skin, hair, or nail is exemplified. The keratinous material to which the treatment method of the present invention is to be applied is preferably skin or hair, and more preferably hair.

The "conditioning effect" in the present invention means a conditioning effect on a keratinous material cleansed by a cleansing agent composition. For example, when the keratinous material is hair, the conditioning effect on the hair refers to an effect of enhancing the lubricity and softness in rinsing, the hard-to-entangle property in rinsing and the rate of drying, of the cleansed hair, and the setting property of the dried hair. The "durability of the conditioning effect" means that the conditioning effect is not lost but retained even when a keratinous material is further cleansed after application of the treatment method of the present invention. In the following description, the effects of the present invention described above will be also collectively referred to as "conditioning effect and durability (enhancing effect) thereof.

[Method for treating keratinous material]

The method for treating a keratinous material of the present invention includes the following Step (I) and Step (II) in this order. Hereinafter, the method for treating a keratinous material of the present invention is also simply referred to as "the treatment method of the present invention".

Step (I): a step of applying a cleansing agent composition (A) that contains an internal olefin sulfonic acid or a salt thereof (a) obtained by sulfonating a starting olefin having an average double bond position of position 3.9 or higher and position 4.5 or lower to a keratinous material Step (II): a step of applying a conditioning agent composition (B) containing a cationic surfactant (b) to the keratinous material

With the above configuration, the treatment method of the present invention imparts a sufficient conditioning effect to the treated keratinous material, and in the cleansed hair, enhances the lubricity and softness in rinsing and also enhances the hard-to-entangle property in rinsing, the rate of drying, and the setting property of dried hair, and furthermore, is superior in the durability of the conditioning effect.

The reason why the above effects are provided by the treatment method of the present invention is not clear but is presumed as follows.

The internal olefin sulfonic acid or a salt thereof (a) (hereinafter also referred to as "component (a)") used in the present invention is obtained by sulfonating a starting olefin having an average double bond position of position 3.9 or higher and position 4.5 or lower, and the cleansing agent composition (A) which contains the component (a) exhibits a superior cleansing ability. When the cleansing agent composition (A) is applied to a keratinous material to cleanse the keratinous material in Step (I), a cleansing effect can be provided and the component (a) remains on the keratinous material surface. Then, when the conditioning agent composition (B) containing the cationic surfactant (b) (hereinafter also referred to as "component (b)") is applied to the keratinous material in Step (II), the component (a) present on the keratinous material surface and the component (b) in the conditioning agent composition (B) ionically interact with each other to form a complex, and the complex remains on the keratinous material surface. It is considered that, since the complex is like a gel which is liable to be hydrated, a superior conditioning effect can be exhibited due to the formation of the complex in the present invention.

<Step (I)>

In Step (I), a cleansing agent composition (A), which contains an internal olefin sulfonic acid or a salt thereof (a) obtained by sulfonating a starting olefin having an average double bond position of position 3.9 or higher and position 4.5 or lower, is applied to a keratinous material. The cleansing agent composition (A) contains the component (a), thereby exhibiting a superior cleansing ability. In addition, the keratinous material is supplied to Step (II) after the cleansing agent composition (A) is applied to the keratinous material in Step (I), thereby enabling the complex formed of the component (a) and the component (b) to adhere on the keratinous material surface to impart a sufficient conditioning effect and a durability thereof to the treated keratinous material.

(Cleansing agent composition (A))

The cleansing agent composition (A) used in Step (I) contains, as the component (a), an internal olefin sulfonic acid or a salt thereof obtained by sulfonating a starting olefin having an average double bond position of position 3.9 or higher and position 4.5 or lower from the viewpoint of exhibiting a superior cleansing ability and the viewpoint of imparting a conditioning effect and durability thereof to the treated keratinous material.

[Internal olefin sulfonic acid or salt thereof (a) obtained by sulfonating starting olefin having an average double bond position of position 3.9 or higher and position 4.5 or lower]

The internal olefin sulfonic acid or a salt thereof which is the component (a) is obtained by sulfonating a starting olefin having an average double bond position of position 3.9 or higher and position 4.5 or lower. That is, the internal olefin sulfonic acid or a salt thereof as the component (a) is a compound obtained by sulfonating a starting olefin having an average double bond position in a specific range used as a starting material, and specifically, a compound obtained by sulfonating the starting olefin, followed by neutralization and hydrolyzation.

Examples of a salt of an internal olefin sulfonic acid include alkali metal salts, such as a sodium salt and a potassium salt; organic amine salts, such as an ammonium salt, a monoethanolamine salt, a diethanolamine salt, a triethanolamine salt, a 2-aminoethanol salt, and a 2-aminomethylpropanediol salt; and basic amino acid salts, such as a lysin salt and an arginine salt. These internal olefin sulfonic acid salts may not necessarily be in the form of salt from the beginning, and a salt produced by a neutralization reaction in the production may be used.

Among them, as the salt of an internal olefin sulfonic acid, from the viewpoint of exhibiting a superior cleansing ability, the viewpoint of imparting a conditioning effect and durability thereof to the treated keratinous material, and the viewpoint of producibility, one or more selected from the group consisting of a sodium salt, a potassium salt, an ammonium salt, and a 2-aminoethanol salt are preferred, one or more selected from the group consisting of a sodium salt and a potassium salt are more preferred, and a sodium salt is further preferred, i.e., a sodium internal olefin sulfonate is further preferred.

The internal olefin sulfonic acid or a salt thereof as the component (a), which is a product obtained from such a starting olefin, is mainly a mixture of a hydroxyalkane sulfonic acid or a salt thereof (hydroxy form, abbreviation "HAS") and an olefin sulfonic acid or a salt thereof (olefin form, abbreviation "IOS").

In the starting olefin which is a starting material of the component (a), the double bond is mainly positioned in the interior of the carbon chain, but sometimes contains a trace amount of an olefin in which the double bond is positioned at position 1 in the carbon chain, i.e., a so-called $\alpha$-olefin. When such a starting olefin is sulfonated, $\beta$-sultone is mainly produced, and a part of the $\beta$-sultone is converted into $\gamma$-sultone and an olefin sulfonic acid, which are further converted into a hydroxyalkane sulfonic acid or a salt thereof and the olefin sulfonic acid or a salt thereof in a neutralization and hydrolyzation step (for example, J. Am. Oil Chem. Soc. 69, 39 (1992)). The hydroxy group of the resulting hydroxyalkane sulfonic acid or a salt thereof is present in the interior of the alkane chain, and the double bond of the olefin sulfonic acid or a salt thereof is present in the interior of the olefin chain.

Accordingly, in this description, these products and mixtures thereof are collectively referred to as an internal olefin sulfonic acid or a salt thereof as the component (a).

From the viewpoint of exhibiting a superior cleansing ability and the viewpoint of imparting a conditioning effect and durability thereof to the treated keratinous material, the number of carbon atoms of the component (a) and the starting olefin is preferably 12 or more, more preferably 14 or more, further preferably 16 or more, and preferably 24 or less, more preferably 22 or less, further preferably 20 or less, furthermore preferably 18 or less. The number of carbon atoms of the component (a) and the starting olefin is preferably 12 to 24, more preferably 14 to 22, further preferably 14 to 20, and furthermore preferably 16 to 18.

The starting olefin that forms the component (a) has an average double bond position of position 3.9 or higher and position 4.5 or lower. The starting olefin having such an average double bond position has a broad distribution of double bonds including position 1 which can be contained in a trace amount.

The positions of double bonds and the distribution thereof in the starting olefin can be found by a measurement using a gas chromatograph mass spectrometer (abbreviation a "GC-MS"). Specifically, the positions of double bonds thereof can be identified by accurately separating components each having different carbon chain lengths and different positions of double bonds with a gas chromatograph spectrometer (hereinafter abbreviated as GC) and subjecting each component to a mass spectrometer (abbreviation "MS"). The positions of double bonds and the distribution thereof are determined from the areas of the GC peaks.

On the other hand, in the component (a) obtained by sulfonating such a starting olefin, the more interior of the carbon chain the sulfonate group introduced by sulfonation is positioned, the more difficult the separation is. Thus, there is currently no established analyzing method. However, it is considerably presumed that the positions of the sulfonate groups in the component (a) almost correspond to the positions of double bonds in the starting olefin and shows a broad distribution without excessive unevenness including position 1. Thus, in the present invention, the component (a) is defined based on the value of the average double bond position in the starting olefin which is a starting material.

In the component (a) used in the present invention, which is an internal olefin sulfonic acid or a salt thereof obtained from a starting olefin having such an average double bond position value as described above, that is, having a broad double bond distribution, the sulfonate groups are present at broad positions without excessive uneven distribution in the carbon chains. In the component (a) obtained from such a starting olefin, the sulfonate groups are present at broad positions without excessive uneven distribution in the carbon chains, and internal olefin sulfonic acids or salts thereof in which the carbon chain from the bonding position of the sulfonate group to the terminal has various lengths are appropriately present together. Accordingly, a good low-temperature storage stability can be exhibited while keeping a superior cleansing effect.

The average double bond position (unit: position) in the starting olefin means the average of the positions of the double bonds in the respective starting olefins present in the entire starting olefin and is determined by the following formula (1).

[Math. 1]

$$\text{Average double bond position of starting olefin} = \sum_{n=1}^{x} n \times C_n / 100 \quad (1)$$

(In the formula (1), x is a value that is y/2 (y is the number of carbon atoms in the starting olefin) when the number of carbon atoms of the starting olefin is an even number, and that is (y-1)/2 when the number of carbon atoms of the starting olefin is an odd number, n represents an integer (unit: position) representing the position of double bond present in each starting olefin. $C_n$ represents the content (unit: % by mass) of the starting olefin in which the double bond is present in the position n in 100% by mass of the entire starting olefin.

From the viewpoint of securing exhibition of a good low-temperature storage stability, the average double bond position in the starting olefin which forms the component (a) is position 3.9 or higher, preferably position 4.0 or higher, and more preferably position 4.1 or higher. From the viewpoint of securing a superior cleansing effect, the average double bond position of the starting olefin is position 4.5 or lower, preferably position 4.4 or lower, and more preferably position 4.3 or lower. The average double bond position of the starting olefin is position 3.9 to position 4.5, preferably position 4.0 to position 4.4, and more preferably position 4.1 to position 4.3.

In the starting olefin, the content of the starting olefin that has a double bond position of position 2 is preferably 10% by mass or more, more preferably 15% by mass or more, further preferably 20% by mass or more, and preferably 35% by mass or less, more preferably 32% by mass or less, further preferably 24% by mass or less. In the starting olefin, the content of the starting olefin that has a double bond position of position 2 is preferably 10 to 35% by mass, more preferably 15 to 32% by mass, and further preferably 20 to 24% by mass.

In the starting olefin, the content of the starting olefin that has a double bond position of position 3 is preferably 10% by mass or more, more preferably 14% by mass or more, further preferably 16% by mass or more, and preferably 30% by mass or less, more preferably 24% by mass or less, further preferably 19% by mass or less. In the starting olefin, the content of the starting olefin that has a double bond position of position 3 is preferably 10 to 30% by mass, more preferably 14 to 24% by mass, and further preferably 16 to 19% by mass.

In the starting olefin, the content of the starting olefin that has a double bond position of position 4 is preferably 10% by mass or more, more preferably 15% by mass or more, further preferably 17% by mass or more, and preferably 30% by mass or less, more preferably 25% by mass or less, further preferably 19% by mass or less. In the starting olefin, the content of the starting olefin that has a double bond position of position 4 is preferably 10 to 30% by mass, more preferably 15 to 25% by mass, and further preferably 17 to 19% by mass.

In the starting olefin, the content of the starting olefin that has a double bond position of position 5 is preferably 5% by mass or more, more preferably 10% by mass or more, further preferably 13% by mass or more, and preferably 25% by mass or less, more preferably 19% by mass or less, further preferably 15% by mass or less. In the starting olefin, the content of the starting olefin that has a double bond position of position 5 is preferably 5 to 25% by mass, more preferably 10 to 19% by mass, and further preferably 13 to 15% by mass.

In the starting olefin, the content of the starting olefin that has a double bond position of position 6 is preferably 5% by mass or more, more preferably 7% by mass or more, further preferably 11% by mass or more, and preferably 20% by mass or less, more preferably 15% by mass or less, further preferably 13% by mass or less. In the starting olefin, the content of the starting olefin that has a double bond position of position 6 is preferably 5 to 20% by mass, more preferably 7 to 15% by mass, and further preferably 11 to 13% by mass.

When the starting olefin contains a starting olefin having 16 or more carbon atoms, the total content of the starting olefins that have a double bond position of position 7 or higher in the starting olefin is preferably 5% by mass or more, more preferably 7% by mass or more, further preferably 12% by mass or more, and preferably 25% by mass or less, more preferably 22% by mass or less, further preferably 16% by mass or less. When the starting olefin contains a starting olefin having 16 or more carbon atoms, the total content of the starting olefins that have a double bond position of position 7 or higher in the starting olefin is preferably 5 to 25% by mass, more preferably 7 to 22% by mass, and further preferably 12 to 16% by mass.

When the starting olefin contains a starting olefin having 16 or more carbon atoms, the mass ratio of the content of the starting olefins that have a double bond position of position 3 to position 5 to the content of the starting olefins that have a double bond position of position 6 to position 8 (starting olefins$_{3-5}$ / starting olefins$_{6-8}$) in the starting olefin is preferably 1.0 or more, more preferably 1.3 or more, further preferably 1.7 or more, and preferably 4.0 or less, more preferably 3.5 or less, further preferably 2.2 or less. When the starting olefin contains a starting olefin having 16 or more carbon atoms, the mass ratio of the content of the starting olefins that have a double bond position of position 3 to position 5 to the content of the starting olefins that have a double bond position of position 6 to position 8 (starting olefins$_{3-5}$ / starting olefins$_{6-8}$) in the starting olefin is preferably 1.0 to 4.0, more preferably 1.3 to 3.5, and further preferably 1.7 to 2.2.

In the starting olefin, the content of the starting olefin ($\alpha$-olefin) that has a double bond position of position 1 which may be inevitably present is preferably less than 5.0% by mass, more preferably less than 3.0% by mass, further preferably less than 2.5% by mass, and furthermore preferably, no $\alpha$-olefin is contained.

The starting olefin can be obtained by isomerizing (transferring the double bond of) the starting olefin that has a double bond position of position 1 ($\alpha$-olefin) which is produced by a dehydration reaction of an alcohol. Specifically, to 100 parts by mass of a 1-alkanol, a solid acid catalyst, such as alumina, is put preferably in an amount of 0.5 parts by mass or more, more preferably 2 parts by mass or more, and preferably 15 parts by mass or less, more preferably 10 parts by mass or less, or is put preferably in an amount of 0.5 to 15 parts by mass, more preferably 2 to 10 parts by mass.

Subsequently, an isomerization reaction is performed with stirring at preferably 220°C or higher, more preferably 260°C or higher, and preferably 350°C or lower, or at preferably 220 to 350°C, more preferably 260 to 350°C, for preferably 1 hour or more, more preferably 3 hours or more, and preferably 30 hours or less, more preferably 10 hours or less, or for preferably 1 to 30 hours, more preferably 3 to 10 hours. The starting olefin described above can be obtained by appropriately distilling the product after completion of the reaction.

The content of the internal olefin sulfonic acids or salts thereof in which the sulfonate group is present at position 1 or higher and position 4 or lower in the component (a) is preferably 40% by mass or more, more preferably 50% by mass or more, further preferably 55% by mass or more, and preferably 75% by mass or less, more preferably 70% by mass or less, further preferably 68% by mass or less. The content of the internal olefin sulfonic acids or salts thereof in which the sulfonate group is present at position 1 or higher and position 4 or lower in the component (a) is preferably 40 to 75% by mass, more preferably 50 to 70% by mass, and further preferably 55 to 68% by mass.

The content of the internal olefin sulfonic acid or a salt thereof in which the sulfonate group is present at position 2 in the component (a) is preferably 10% by mass or more, more preferably 13% by mass or more, further preferably 17% by mass or more, and preferably 35% by mass or less, more preferably 30% by mass or less, further preferably 25% by mass or less. The content of the internal olefin sulfonic acid or a salt thereof in which the sulfonate group is present at position 2 in the component (a) is preferably 10 to 35% by mass, more preferably 13 to 30% by mass, and further preferably 17 to 25% by mass.

The content of the internal olefin sulfonic acid or a salt thereof in which the sulfonate group is present at position 3 in the component (a) is preferably 5% by mass or more, more preferably 11% by mass or more, further preferably 15% by mass or more, and preferably 30% by mass or less, more preferably 25% by mass or less, further preferably 20% by mass or less. The content of the internal olefin sulfonic acid or a salt thereof in which the sulfonate group is present at position 3 in the component (a) is preferably 5 to 30% by mass, more preferably 11 to 25% by mass, and further preferably 15 to 20% by mass.

The content of the internal olefin sulfonic acid or a salt thereof in which the sulfonate group is present in position 4 in the component (a) is preferably 15% by mass or more, more preferably 18% by mass or more, further preferably 19% by mass or more, and preferably 30% by mass or less, more preferably 25% by mass or less, further preferably 23% by mass or less. The content of the internal olefin sulfonic acid or a salt thereof in which the sulfonate group is present at position 4 in the component (a) is preferably 15 to 30% by mass, more preferably 18 to 25% by mass, and further preferably 19 to 23% by mass.

The content of the internal olefin sulfonic acid or a salt thereof in which the sulfonate group is present at position 1 in the component (a) is preferably less than 5.0% by mass, more preferably less than 3.0% by mass, further preferably less than 2.5% by mass, and further preferably, the internal olefin sulfonic acid or a salt thereof in which the sulfonate group is present in position 1 is not contained.

The mass ratio of the content of the hydroxy form (HAS) to the content of the olefin form (IOS) (hydroxy form /olefin form) in the component (a) is, from the viewpoints of enhancing the productivity and reducing impurities, preferably 50/50 to 100/0, more preferably 60/40 to 100/0, further preferably 70/30 to 100/0, furthermore preferably 75/25 to 100/0, and furthermore preferably 75/25 to 95/5.

The mass ratio (hydroxy form / olefin form) is determined based on the areas of HPLC-MS peaks obtained by separating the hydroxy form and the olefin form from the component (a) by HPLC and subjecting each form to MS.

Since the component (a) is obtained by sulfonating a starting olefin, the unreacted starting olefin and inorganic compounds possibly remain in the component (a). Less contents of the components are preferred.

The content of the unreacted starting olefin in the component (a) is preferably less than 5.0% by mass, more preferably less than 3.0% by mass, further preferably less than 1.5% by mass, and furthermore preferably less than 1.0% by mass.

The content of inorganic compounds in the component (a) is preferably less than 7.5% by mass, more preferably less than 5.0% by mass, further preferably less than 3.0% by mass, furthermore preferably less than 2.0% by mass, and furthermore preferably less than 1.6% by mass.

The component (a) is obtained by sulfonating the starting olefin by a reaction with sulfur trioxide, and specifically, the starting olefin is sulfonated, followed by neutralization and hydrolysis.

More specifically, the amount of sulfur trioxide used in sulfonating the starting olefin is, from the viewpoint of enhancing the yield of the component (a) and the viewpoint of enhancing the reactivity, relative to one mole of the starting olefin, preferably 0.8 moles or more, more preferably 0.9 moles or more, and further preferably 0.95 moles or more. In addition, the amount of sulfur trioxide used is, from the viewpoint of economy and the viewpoint of suppressing unwanted coloring of the component (a), preferably 1.2 moles or less, more preferably 1.1 moles or less, and further preferably 1.05 moles or less. The amount of sulfur trioxide used relative to 1 mole of the starting olefin is preferably 0.8 to 1.2 moles, more preferably 0.9 to 1.1 moles, and more preferably 0.95 to 1.05 moles.

The reaction temperature in sulfonating the starting olefin is, from the viewpoint of preventing coagulation of sulfur trioxide and the component (a), preferably 0°C or higher, and from the viewpoint of suppressing unwanted coloring of the component (a), preferably 50°C or lower. The reaction temperature in sulfonating the starting olefin is preferably 0 to 50°C.

In neutralization, an alkali compound, such as sodium hydroxide, potassium hydroxide, ammonia, or 2-aminoethanol, is reacted. Among them, from the viewpoint of easily obtaining a component (a) that is a sodium salt, sodium hydroxide is preferred. The amount of such an alkali compound added is, from the viewpoint of suppressing production of impurities, such as the starting olefin and an inorganic salt, and the viewpoint of enhancing the reactivity, preferably 1 mole or more, more preferably 1.03 moles or more relative to 1 mole of sulfonate group. The amount of the alkali compound added is, from the viewpoint of economy and the viewpoint of suppressing production of impurities, such as the starting olefin and an inorganic salt, preferably 2.5 moles or less, more preferably 2.0 moles or less, and further preferably 1.5 moles or less. The amount of the alkali compound added relative to 1 mole of sulfonate group is preferably 1.00 to 2.5 moles, more preferably 1.03 to 2.0 moles, and furthermore preferably 1.03 to 1.5 moles.

In neutralization, the temperature in mixing the sulfonated starting olefin and the alkali compound and the reaction temperature are, from the viewpoint of suppressing production of impurities, such as the internal olefin and an inorganic salt, by side reactions, preferably 40°C or lower, more preferably 35°C or lower, and from the viewpoint of enhancing the reactivity, preferably 0°C or higher, more preferably 10°C or higher, further preferably 15°C or higher, furthermore preferably 20°C or higher. The temperature in mixing the sulfonated starting olefin and the alkali compound and the reaction temperature are preferably 0 to 40°C, more preferably 10 to 35°C, further preferably 15 to 35°C, and furthermore preferably 20 to 35°C.

The reaction temperature in the hydrolysis performed after the neutralization is, from the viewpoint of enhancing the reactivity in the presence of water, preferably 120°C or higher, more preferably 140°C or higher, and further preferably 160°C or higher. In addition, the reaction temperature in the hydrolysis is, from the viewpoint of suppressing decomposition of the product, preferably 220°C or lower, more preferably 180°C or lower. The reaction temperature in the hydrolysis is preferably 120 to 220°C, more preferably 140 to 180°C, and further preferably 160 to 180°C.

The reaction time in the hydrolysis is, from the viewpoint of completing the reaction, preferably 30 minutes or more, and more preferably 45 minutes or more. The reaction time in the hydrolysis is, from the viewpoint of enhancing the productivity, preferably 240 minutes or less, more preferably 180 minutes or less, further preferably 120 minutes or less, and furthermore preferably 90 minutes or less. The reaction time in the hydrolysis is preferably 30 to 240 minutes, more preferably 45 to 180 minutes, furthermore preferably 45 to 120 minutes, and furthermore preferably 45 to 90 minutes. The reactions can be continuously performed. After completion of the reaction, purification by extraction, washing, or the like can be performed.

The content of the component (a) in the cleansing agent composition (A) is, from the viewpoint of exhibiting a superior cleansing ability and the viewpoint of imparting a conditioning effect and durability thereof to the treated keratinous material, preferably 0.1% by mass or more, more preferably 0.2% by mass or more, further preferably 0.3% by mass or more, furthermore preferably 0.5% by mass or more, furthermore preferably 1.0% by mass or more, furthermore preferably 2.0% by mass or more, furthermore preferably 5.0% by mass or more, furthermore preferably 8.0% by mass or more, and preferably 30% by mass or less, more preferably 25% by mass or less, further preferably 20% by mass or less, furthermore preferably 18% by mass or less, furthermore preferably 14% by mass or less. The content of the component (a) in the cleansing agent composition (A) is preferably 0.1 to 30% by mass, more preferably 0.2 to 30% by mass, further preferably 0.3 to 25% by mass, furthermore preferably 0.5 to 25% by mass, furthermore preferably 1.0 to 20% by mass, furthermore preferably 2.0 to 20% by mass, furthermore preferably 5.0 to 18% by mass, and furthermore preferably 8.0 to 14% by mass.

[Cationic polymer]

The cleansing agent composition (A) can contain a cationic polymer from the viewpoint of more improving the feeling of treated hair. As used herein, the "cationic polymer" means a polymer having a cationic group or a group that provides a cationic property when being dissolved in water.

Examples of the cationic polymer include one or more selected from the group consisting of a cationized polygalactomannan, a cationized hydroxyalkyl cellulose, a diallyl quaternary ammonium salt polymer, a copolymer containing methacrylamidepropyltrimethylammonium chloride, and a crosslinked cationic polymer.

Examples of the cationized polygalactomannan include one or more selected from the group consisting of a cationized guar gum, a cationized tara gum, and a cationized locust bean gum.

Examples of the cationized hydroxyalkyl cellulose include one or more selected from the group consisting of a cationized hydroxyethyl cellulose and a cationized hydroxypropyl cellulose.

Examples of the diallyl quaternary ammonium salt polymer include polydiallyldimethylammonium chloride, a diallyldimethylammonium chloride / acrylic acid copolymer, a diallyldimethylammonium chloride / acrylamide copolymer, and a diallyldimethylammonium chloride /acrylic acid /acrylamide copolymer.

Examples of the copolymer containing methacrylamidepropyltrimethylammonium chloride include one or more selected from the group consisting of an acrylic acid /methyl acrylate /methacrylamidepropyltrimethylammonium chloride copolymer and an acrylic acid / acrylamide / methacrylamidepropyltrimethylammonium chloride copolymer.

An example of the crosslinked cationic polymer is an N,N-dimethylaminoethyl methacrylic acid diethylsulfuric acid salt / N,N-dimethylacrylamide / dimethacrylic acid polyethylene glycol copolymer.

Among the above compounds, as the cationic polymer, from the viewpoint of more improving the feeling of treated hair, one or more selected from the group consisting of a cationized hydroxyalkyl cellulose and a crosslinked cationic polymer are preferred, a cationized hydroxyalkyl cellulose is more preferred, and a cationized hydroxyethyl cellulose is further preferred.

When the cleansing agent composition (A) contains a cationic polymer, the content of the cationic polymer in the cleansing agent composition (A) is, from the viewpoint of improving the feeling of treated hair, preferably 0.01% by mass or more, more preferably 0.05% by mass or more, further preferably 0.1% by mass or more, and from the viewpoint of handleability of the cleansing agent composition (A), preferably 10% by mass or less, more preferably 3% by mass or less, further preferably 1% by mass or less. The content of the cationic polymer in the cleansing agent composition (A) is preferably 0.01 to 10% by mass, more preferably 0.05 to 3% by mass, and further preferably 0.1 to 1% by mass.

[Electrolyte]

From the viewpoint of allowing the component (a) to effectively adsorb on the keratinous material surface to impart a high conditioning effect and durability thereof to the treated keratinous material, the cleansing agent composition (A) preferably further contains an electrolyte. It is considered that, when the cleansing agent composition (A) containing an electrolyte is applied onto a keratinous material and the keratinous material is then washed with water, the concentration of electrolytes in the system decreases and the component (a) is liable to adsorb on the keratinous material surface.

In the present invention, the "electrolyte" means a salt compound that is ionically dissociated in water.

Examples of the electrolyte include sodium chloride, potassium chloride, magnesium chloride, sodium citrate, potassium benzoate, ammonium chloride, sodium carbonate, dipotassium phosphate, and monoethanolamine sulfate. One of the electrolytes can be used alone or two or more thereof can be used in combination.

Among the above electrolytes, from the viewpoint of solubility in water, the viewpoint of allowing the complex formed from the component (a) and the component (b) to effectively adsorb on the keratinous material surface to impart a high conditioning effect and durability thereof to the treated keratinous material, and the viewpoint of economy, the electrolyte is preferably an inorganic salt, more preferably one or more selected from the group consisting of sodium chloride, potassium chloride, and magnesium chloride, further preferably one or more selected from the group consisting of sodium chloride and potassium chloride, and furthermore preferably sodium chloride.

When the cleansing agent composition (A) contains an electrolyte, the content of the electrolyte in the cleansing agent composition (A) is, from the viewpoint of allowing the component (a) to more effectively adsorb on the keratinous material surface to impart a high conditioning effect and durability thereof to the treated keratinous material, preferably 0.1% by mass or more, more preferably 0.2% by mass or more, further preferably 0.3% by mass or more, and preferably 10% by mass or less, more preferably 5% by mass or less, further preferably 3% by mass or less, furthermore preferably 1% by mass or less. The content of the electrolyte in cleansing agent composition (A) is preferably 0.1 to 10% by mass, more preferably 0.1 to 5% by mass, further preferably 0.2 to 3% by mass, and furthermore preferably 0.3 to 1% by mass.

[Aqueous medium]

From the viewpoint of dispersing the component (a) and the viewpoint of adjusting the cleansing agent composition (A) into a desired dosage form to enhance the handleability, the cleansing agent composition (A) preferably contains an aqueous medium.

Examples of the aqueous medium include water; lower alcohols, such as ethanol and isopropyl alcohol; and low molecular diols and triols having 6 or less carbon atoms, such as 1,3-butylene glycol, glycerol, ethylene glycol, propylene glycol, and dipropylene glycol. One of the aqueous mediums may be used alone or two or more thereof may be used in combination. Among them, from the viewpoint of capability of also acting as a medium in sulfonating the starting olefin to obtain the component (a), the aqueous medium is preferably water.

When the cleansing agent composition (A) contains an aqueous medium, from the viewpoint of dispersing the component (a) and the viewpoint of adjusting the cleansing agent composition (A) into a desired dosage form to enhance the handleability, the content of the aqueous medium in the cleansing agent composition (A) is preferably 30% by mass or more, more preferably 50% by mass or more, further preferably 70% by mass or more, furthermore preferably 80% by mass or more, and preferably 99.4% by mass or less.

[Other components]

The cleansing agent composition (A) can further contain other components, such as a pH modifier, a surfactant other than the component (a), an antioxidant, a higher alcohol, an oil agent, an aromatic alcohol, a vitamin agent, a bactericide, an anti-inflammatory agent, an anti-dandruff agent, a preservative, a chelating agent, a humectant, a pearl agent, a ceramide, a fragrance, and an UV absorber.

(Method for producing cleansing agent composition (A))

The cleansing agent composition (A) can be produced by an ordinary method. For example, the cleansing agent composition (A) can be produced by blending the component (a) and other components used as required and mixing the blend with a known stirring apparatus or the like.

(Method for applying cleansing agent composition (A))

In Step (I), examples of a method for applying the cleansing agent composition (A) to a keratinous material include a method in which the cleansing agent composition (A) is put-spread, sprayed, or flow-spread onto the keratinous material; and a method in which the keratinous material is immersed in the cleansing agent composition (A). Among them, a method in which the cleansing agent composition (A) is put-spread on the keratinous material is preferred.

The keratinous material to which the cleansing agent composition (A) is applied may be dry or may be wet.

The amount of the cleansing agent composition (A) applied on the keratinous material is not particularly limited. For example, when the keratinous material is hair, from the viewpoint of the hair cleansing ability, the bath ratio of the cleansing agent composition (A) to the dry mass of the hair to be treated (mass of cleansing agent composition (A) / dry mass of hair to be treated) is preferably 0.005 or more, more preferably 0.01 or more, further preferably 0.05 or more, and preferably 20 or less, more preferably 15 or less, further preferably 12 or less. The bath ratio of the cleansing agent composition (A) to the dry mass of the hair to be treated (mass of cleansing agent composition (A) / dry mass of hair to be treated) is preferably 0.005 to 20, more preferably 0.01 to 15, and further preferably 0.05 to 12.

The hair to which the cleansing agent composition (A) is applied may be all of the hair of a head or may be a part thereof.

After the cleansing agent composition (A) is applied to the keratinous material, a cleansing operation in which, for example, the cleansing agent composition (A) is spread to the keratinous material surface and is adapted thereto, or the cleansing agent composition (A) is foamed on the keratinous material surface, is preferably performed depending on the type and site of the keratinous material.

After the cleansing agent composition (A) is applied on the keratinous material or after the cleansing operation is performed, a step of washing away the cleansing agent composition with water, hot water, or the like is preferably performed.

The keratinous material after washing away the cleansing agent composition (A) may be subjected to Step (II) as it is without drying, or may be supplied to Step (II) after drying once. In the case of hair, the hair is dried by, for example, towel drying, natural dry, or forced dry with a dryer or the like. The drying methods may be combined.

<Step (II) >

In Step (II), the conditioning agent composition (B) containing a cationic surfactant (b) is applied on the keratinous material. In the treatment method of the present invention, a conditioning effect and durability thereof can be imparted to the treated keratinous material by performing Step (I) and Step (II) in this order.

(Conditioning agent composition (B))

From the viewpoint of imparting a conditioning effect and durability thereof to the treated keratinous material, the conditioning agent composition (B) used in Step (II) contains a cationic surfactant as a component (b).

[Cationic surfactant (b)]

Examples of the cationic surfactant (b) used in the conditioning agent composition (B) (hereinafter also referred to as "component (b)") include (i) an alkyltrimethylammonium salt, (ii) an alkoxyalkyltrimethylammonium salt, (iii) a dialkyldimethylammonium salt, (iv) an alkylamidealkyltrimethylammonium salt, (v) an alkyldimethylamine and a salt thereof, (vi) an alkoxyalkyldimethylamine and a salt thereof, and (vii) an alkylamidealkyldimethylamine and a salt thereof. One or two or more of the cationic surfactants can be used.

An example of the alkyltrimethylammonium salt (i) is an alkyltrimethylammonium salt having an alkyl group preferably having 12 or more and 22 or less carbon atoms, more preferably having 16 or more and 20 or less carbon atoms, and specific examples thereof include cetyltrimethylammonium chloride (cetrimonium chloride), stearyltrimethylammonium chloride (steartrimonium chloride), and behenyltrimethylammonium chloride.

An example of the alkoxyalkyltrimethylammonium salt (ii) is an alkoxyalkyltrimethylammonium salt having an alkoxy group preferably having 12 or more and 22 or less carbon atoms, more preferably having 16 or more and 20 or less carbon atoms, and specific examples thereof include stearoxypropyltrimethylammonium chloride, stearoxyethyltrimethylammonium chloride, and stearoxyhydroxypropyltrimethylammonium chloride.

An example of the dialkyldimethylammonium salt (iii) is a dialkyldimethylammonium salt having an alkyl group preferably having 12 or more and 22 or less carbon atoms, more preferably having 16 or more and 20 or less carbon atoms, and specific examples thereof include distearyldimethylammonium chloride.

An example of the alkylamidealkyltrimethylammonium salt (iv) is an alkylamidealkyltrimethylammonium salt in which the alkyl group in the alkylamide moiety preferably has 11 or more and 21 or less carbon atoms, more preferably has 13 or more and 19 or less carbon atoms, and specific examples thereof include palmitamidepropyltrimethylammonium chloride (palmitamidepropyltrimonium chloride).

The alkyldimethylamine (v), the alkoxyalkyldimethylamine (vi), and the alkylamidealkyldimethylamine (vii) react with an acid to produce a tertiary amine salt and then produce a cationic surfactant.

The alkyl group in the alkyldimethylamine and a salt thereof (v) and the alkoxy group in the alkoxyalkyldimethylamine and a salt thereof (vi) preferably have 12 or more and 22 or less carbon atoms, more preferably has 16 or more and 20 or less carbon atoms.

The alkyl group in the alkylamide moiety of the alkylamidealkyldimethylamine and a salt thereof (vii) preferably have 11 or more and 21 or less carbon atoms, more preferably have 15 or more and 19 or less carbon atoms.

The amines (v) to (vii) may be previously reacted with an acid to produce a salt, which may be blended into the conditioning agent composition (B). Alternatively, the amines (v) to (vii) may be blended into the conditioning agent composition (B) as it is in the form of amine and a salt is formed in the composition by blending an acid into the conditioning agent composition (B). Accordingly, the amines and salts thereof are herein defined as a cationic surfactant. The content thereof is calculated based on the mass of the amine.

Examples of the salt of the amines (v) to (vii) include salts with an organic acid or an inorganic acid. Examples of the organic acid include monocarboxylic acids, such as acetic acid and propionic acid; dicarboxylic acids, such as malonic acid, succinic acid, glutaric acid, adipic acid, maleic acid, fumaric acid, and phthalic acid; a polycarboxylic acid, such as polyglutamic acid; hydroxycarboxylic acids, such as glycolic acid, lactic acid, hydroxyacrylic acid, glyceric acid, malic acid, tartaric acid, and citric acid; and acidic amino acids, such as glutamic acid and aspartic acid. Examples of the inorganic acid include hydrochloric acid, sulfuric acid, and phosphoric acid. Among them, an organic acid is preferred, and one or more selected from the group consisting of a dicarboxylic acid, a hydroxycarboxylic acid, and an acidic amino acid are more preferred. As the dicarboxylic acid, one or more selected from the group consisting of maleic acid and succinic acid are more preferred. As the hydroxycarboxylic acid, one or more selected from the group consisting of glycolic acid, lactic acid, and malic acid are more preferred. As the acidic amino acid, glutamic acid is more preferred.

Examples of the alkyldimethylamine and a salt thereof (v) include N,N-dimethylbehenylamine, N,N-dimethylstearylamine, and an organic acid salt thereof, and N,N-dimethylbehenylamine lactic acid salt, N,N-dimethylstearylamine glycolic acid salt, and the like are preferred.

Examples of the alkoxyalkyldimethylamine and a salt thereof (vi) include N,N-dimethyl-3-hexadecyloxypropylamine, N,N-dimethyl-3-octadecyloxypropylamine, and an organic acid salt thereof. N,N-dimethyl-3-hexadecyloxypropylamine or a salt thereof and N,N-dimethyl-3-octadecyloxypropylamine (stearoxypropyldimethylamine) or a salt thereof are preferred.

Examples of the alkylamidealkyldimethylamine and a salt thereof (vii) include N-[3-(dimethylamino)propyl]docosanamide, N-[3-(dimethylamino)propyl]stearamide, and an organic acid salt thereof. N-[3-(dimethylamino)propyl]docosanamide lactic acid salt and N-[3-(dimethylamino)propyl]stearamide glycolic acid salt are preferred.

Among the above compounds, from the viewpoint of imparting a conditioning effect and durability thereof to the treated keratinous material, the cationic surfactant (b) is preferably one or more selected from the group consisting of the alkyltrimethylammonium salt (i) and the alkoxyalkyldimethylamine and a salt thereof (vi), more preferably one or more selected from the group consisting of cetyltrimethylammonium chloride (cetrimonium chloride), stearyltrimethylammonium chloride (steartrimonium chloride), behenyltrimethylammonium chloride, and N,N-dimethyl-3-octadecyloxypropylamine and a salt thereof.

The content of the component (b) in conditioning agent composition (B) is, from the viewpoint of imparting a conditioning effect and durability thereof to the treated keratinous material, preferably 0.1% by mass or more, more preferably 0.5% by mass or more, further preferably 1.0% by mass or more, furthermore preferably 2.0% by mass or more, furthermore preferably 3.0% by mass or more, and preferably 25% by mass or less, more preferably 20% by mass or less, further preferably 15% by mass or less, furthermore preferably 10% by mass or less. The content of the component (b) in the conditioning agent composition (B) is preferably 0.1 to 25% by mass, more preferably 0.5 to 20% by mass, further preferably 1.0 to 20% by mass, furthermore preferably 2.0 to 15% by mass, furthermore preferably 2.0 to 10% by mass, and furthermore preferably 3.0 to 10% by mass.

[Higher alcohol]

From the viewpoint of more improving the feeling of treated hair, the conditioning agent composition (B) can contain a higher alcohol.

The number of carbon atoms of the higher alcohol is preferably 12 or more and 22 or less. Examples of the higher alcohol include cetyl alcohol, oleyl alcohol, stearyl alcohol, isostearyl alcohol, 2-octyldodecanol, myristyl alcohol, behenyl alcohol, and cetostearyl alcohol. One of the higher alcohols can be used alone or two or more thereof can be used in combination.

When the conditioning agent composition (B) contains a higher alcohol, the content of the higher alcohol in the conditioning agent composition (B) is, from the viewpoint of more improving the feeling of treated hair, preferably 1.0% by mass or more, more preferably 2.0% by mass or more, further preferably 3.0% by mass or more, and preferably 15% by mass or less, more preferably 12% by mass or less, further preferably 10% by mass or less. The content of the higher alcohol the conditioning agent composition (B) is preferably 1.0 to 15% by mass, more preferably 2.0 to 12% by mass, and further preferably 3.0 to 10% by mass.

[Aqueous medium]

From the viewpoint of dispersing the component (b) and the viewpoint of adjusting the conditioning agent composition (B) into a desired dosage form to enhance the handleability, the conditioning agent composition (B) preferably contains an aqueous medium.

Examples of the aqueous medium include water; lower alcohols, such as ethanol and isopropyl alcohol; and low molecular diols and triols having 6 or less carbon atoms, such as 1,3-butylene glycol, glycerol, ethylene glycol, propylene glycol, and dipropylene glycol. One of the aqueous mediums may be used alone or two or more thereof may be used in combination. Among them, the aqueous medium is preferably water.

When the conditioning agent composition (B) contains an aqueous medium, from the viewpoint of dispersing the component (b) and the viewpoint of adjusting the conditioning agent composition (B) into a desired dosage form to enhance the handleability, the content of the aqueous medium in the conditioning agent composition (B) is preferably 30% by mass or more, more preferably 50% by mass or more, further preferably 70% by mass or more, furthermore preferably 75% by mass or more, and preferably 99.9% by mass or less, more preferably 95% by mass or less.

[Other components]

The conditioning agent composition (B) can further contain other components, such as the organic acid or inorganic acid or another pH modifier, a surfactant other than the component (b), an antioxidant, an oil agent, an aromatic alcohol, a vitamin agent, a bactericide, an anti-inflammatory agent, an anti-dandruff agent, a preservative, a chelating agent, a humectant, a pearl agent, a ceramide, a fragrance, and an UV absorber.

(Method for producing conditioning agent composition (B))

The conditioning agent composition (B) can be produced by an ordinary method. For example, the conditioning agent composition (B) can be produced by blending the component (b) and other components used as required and mixing the blend with a known stirring apparatus or the like.

(Method for applying conditioning agent composition (B))

Examples of a method for applying the conditioning agent composition (B) to a keratinous material in Step (II) include a method in which the conditioning agent composition (B) is put-spread, sprayed, or flow-spread onto the keratinous material; and a method in which the keratinous material is immersed in the conditioning agent composition (B). Among them, a method in which the conditioning agent composition (B) is put-spread on the keratinous material is preferred.

The keratinous material to which the conditioning agent composition (B) is applied may be dry or may be wet, but from the viewpoint of subjecting the keratinous material treated in Step (I) to Step (II) as it is and the viewpoint of efficiently forming a complex to impart a conditioning effect and durability thereof to the treated keratinous material, the conditioning agent composition (B) is preferably applied to a keratinous material in a wet state.

The amount of the conditioning agent composition (B) applied to a keratinous material is not particularly limited. For example, when the keratinous material is hair, from the viewpoint of enhancing the conditioning effect and durability thereof, the bath ratio of the conditioning agent composition (B) to the dry mass of the hair to be treated (mass of conditioning agent composition (B) / dry mass of hair to be treated) is preferably 0.005 or more, more preferably 0.01 or more, further preferably 0.05 or more, and preferably 20 or less, more preferably 15 or less, further preferably 12 or less. The bath ratio of the conditioning agent composition (B) to the dry mass of the hair to be treated (mass of conditioning agent composition (B) / dry mass of hair to be treated) is preferably 0.005 to 20, more preferably 0.01 to 15, and further preferably 0.05 to 12.

After the conditioning agent composition (B) is applied to the keratinous material, a step of adapting the conditioning agent composition (B) to the keratinous material surface and then washing away the conditioning agent composition (B) with water, hot water, or the like is preferably performed.

From the viewpoint of enhancing the conditioning effect and durability thereof, after applying the conditioning agent composition (B) to a keratinous material and before washing away the conditioning agent composition (B), a step of leaving the keratinous material as it is in the state with the conditioning agent composition (B) put-spread thereon may be performed. The time for leaving is preferably 3 minutes or more, more preferably 5 minutes or more. From the viewpoint of treatment efficiency, the time for leaving is preferably 30 minutes or less.

In the treatment method of the present invention, Step (I) and Step (II) are simply performed in this order, and a series of treatments of the cleansing by Step (I) and the conditioning by Step (II) is preferably repeated. This is because, when the series of treatments of Step (I) and Step (II) is repeated, the complex formed of the component (a) and the component (b) is deposited on the keratinous material surface, and thus, the conditioning effect and durability thereof is more liable to increase. The number of repetitions of the series of treatments of Step (I) and Step (II) is not particularly limited, and generally is twice or more, and from the viewpoint of enhancing the conditioning effect and durability thereof and the viewpoint of simpleness of the treatment, the number of repetitions is preferably three times or more, more preferably five times or more.

[Cosmetic kit for treating keratinous material]

The present invention provides a cosmetic kit for treating a keratinous material (hereinafter also referred to simply as "cosmetic kit"), the kit including a cleansing agent composition (A) containing an internal olefin sulfonic acid or a salt thereof (a) obtained by sulfonating a starting olefin having an average double bond position of position 3.9 or higher and position 4.5 or lower and a conditioning agent composition (B) containing a cationic surfactant (b).

The cosmetic kit of the present invention can impart a conditioning effect and durability thereof to the keratinous material treated by applying a cleansing agent composition (A) and a conditioning agent composition (B) in this order to a keratinous material.

The cleansing agent composition (A) and the conditioning agent composition (B) which constitute the cosmetic kit of the present invention and suitable aspects thereof are the same as described above. The cleansing agent composition (A) and the conditioning agent composition (B) each can be applied to a keratinous material by the same method as the treatment method of the present invention.

[Composition for treating a keratinous material]

The present invention also provides a composition for treating a keratinous material, the composition being to be applied to a keratinous material before application of a conditioning agent composition (B) containing a cationic surfactant (b), the composition containing an internal olefin sulfonic acid or a salt thereof (a) obtained by sulfonating a starting olefin having an average double bond position of position 3.9 or higher and position 4.5 or lower.

By applying the composition for treating a keratinous material of the present invention to a keratinous material before application of the conditioning agent composition (B), it is possible to form a complex of the component (a) and the component (b) on the keratinous material surface to impart a conditioning effect and durability thereof to the treated keratinous material. The component (a), the conditioning agent composition (B), and suitable aspects thereof are the same as described above.

The composition for treating a keratinous material containing the component (a) is simply a composition that is to be applied to a keratinous material before application of a conditioning agent composition (B) and may be a cleansing agent composition. Other examples of the composition for treating a keratinous material include a rinse agent composition, a conditioning agent composition, and a treatment agent composition, for a keratinous material. From the viewpoint of enhancing the conditioning effect and durability thereof, the composition for treating a keratinous material is preferably a cleansing agent composition, and more preferably the cleansing agent composition (A).

Hereinunder, the present invention further discloses the following aspects with respect to the embodiments described above.

<1> A method for treating a keratinous material, including Step (I) and Step (II) in this order:

Step (I): a step of applying a cleansing agent composition (A) that contains an internal olefin sulfonic acid or a salt thereof (a) obtained by sulfonating a starting olefin having an average double bond position of position 3.9 or higher and position 4.5 or lower to a keratinous material;
Step (II): a step of applying a conditioning agent composition (B) containing a cationic surfactant (b) to the keratinous material.

<2> The method for treating a keratinous material according to <1>, in which the component (a) has preferably 12 to 24 carbon atoms, more preferably 14 to 22 carbon atoms, further preferably 14 to 20 carbon atoms, furthermore preferably 16 to 18 carbon atoms.
<3> The method for treating a keratinous material according to <1> or <2>, in which the starting olefin has an average double bond position of preferably position 4.0 to position 4.4, and more preferably position 4.1 to position 4.3.
<4> The method for treating a keratinous material according to any one of <1> to <3>, in which the starting olefin has a content of a starting olefin that has a double bond position of position 2 of preferably 10 to 35% by mass, more preferably 15 to 32% by mass, and further preferably 20 to 24% by mass.
<5> The method for treating a keratinous material according to any one of <1> to <4>, in which the starting olefin has a content of a starting olefin that has a double bond position of position 3 of preferably 10 to 30% by mass, more preferably 14 to 24% by mass, and further preferably 16 to 19% by mass.
<6> The method for treating a keratinous material according to any one of <1> to <5>, in which the starting olefin has a content of a starting olefin that has a double bond position of position 4 of preferably 10 to 30% by mass, more preferably 15 to 25% by mass, and further preferably 17 to 19% by mass.
<7> The method for treating a keratinous material according to any one of <1> to <6>, in which the starting olefin has a content of a starting olefin that has a double bond position of position 5 of preferably 5 to 25% by mass, more preferably 10 to 19% by mass, and further preferably 13 to 15% by mass.
<8> The method for treating a keratinous material according to any one of <1> to <7>, in which the starting olefin has a content of a starting olefin that has a double bond position of position 6 of preferably 5 to 20% by mass, more preferably 7 to 15% by mass, and further preferably 11 to 13% by mass.
<9> The method for treating a keratinous material according to any one of <1> to <8>, in which when the starting olefin contains a starting olefin having 16 or more carbon atoms, the starting olefin has a total content of starting olefins that have a double bond position of position 7 or higher of preferably 5 to 25% by mass, more preferably 7 to 22% by mass, and further preferably 12 to 16% by mass.

<10> The method for treating a keratinous material according to any one of <1> to <9>, in which when the starting olefin contains a starting olefin having 16 or more carbon atoms, the starting olefin has a mass ratio of a content of starting olefins that have a double bond position of position 3 to position 5 to the content of starting olefins that have a double bond position of position 6 to position 8 (starting olefins$_{3-5}$ / starting olefins$_{6-8}$) of preferably 1.0 to 4.0, more preferably 1.3 to 3.5, and further preferably 1.7 to 2.2.

<11> The method for treating a keratinous material according to any one of <1> to <10>, in which the starting olefin has a content of a starting olefin that has a double bond position of position 1 ($\alpha$-olefin) of preferably less than 5.0% by mass, more preferably less than 3.0% by mass, further preferably less than 2.5% by mass, and furthermore preferably does not contain $\alpha$-olefin.

<12> The method for treating a keratinous material according to any one of <1> to <11>, in which the component (a) has a content of internal olefin sulfonic acids or salts thereof in which the sulfonate group is present at position 1 or higher and 4 or lower of preferably 40 to 75% by mass, more preferably 50 to 70% by mass, and further preferably 55 to 68% by mass.

<13> The method for treating a keratinous material according to any one of <1> to <12>, in which the component (a) has a content of an internal olefin sulfonic acid or a salt thereof in which the sulfonate group is present at position 2 of preferably 10 to 35% by mass, more preferably 13 to 30% by mass, and further preferably 17 to 25% by mass.

<14> The method for treating a keratinous material according to any one of <1> to <13>, in which the component (a) has a content of an internal olefin sulfonic acid or a salt thereof in which the sulfonate group is present at position 3 of preferably 5 to 30% by mass, more preferably 11 to 25% by mass, and further preferably 15 to 20% by mass.

<15> The method for treating a keratinous material according to any one of <1> to <14>, in which the component (a) has a content of an internal olefin sulfonic acid or a salt thereof in which the sulfonate group is present at position 4 of preferably 15 to 30% by mass, more preferably 18 to 25% by mass, and further preferably 19 to 23% by mass.

<16> The method for treating a keratinous material according to any one of <1> to <15>, in which the component (a) has a content of an internal olefin sulfonic acid or a salt thereof in which the sulfonate group is present at position 1 of preferably less than 5.0% by mass, more preferably less than 3.0% by mass, further preferably less than 2.5% by mass, and furthermore preferably does not contain an internal olefin sulfonic acid or a salt thereof in which the sulfonate group is present at position 1.

<17> The method for treating a keratinous material according to any one of <1> to <16>, in which the component (a) has a mass ratio of the content of the hydroxy form (HAS) to the content of the olefin form (IOS) (hydroxy form / olefin form) of preferably 50/50 to 100/0, more preferably 60/40 to 100/0, further preferably 70/30 to 100/0, furthermore preferably 75/25 to 100/0, and furthermore preferably 75/25 to 95/5.

<18> The method for treating a keratinous material according to any one of <1> to <17>, in which the cleansing agent composition (A) has a content of the component (a) of preferably 0.1 to 30% by mass, more preferably 0.2 to 30% by mass, further preferably 0.3 to 25% by mass, furthermore preferably 0.5 to 25% by mass, furthermore preferably 1.0 to 20% by mass, furthermore preferably 2.0 to 20% by mass, furthermore preferably 5.0 to 18% by mass, and furthermore preferably 8.0 to 14% by mass.

<19> The method for treating a keratinous material according to any one of <1> to <18>, in which the cleansing agent composition (A) contains a cationic polymer, preferably one or more selected from the group consisting of a cationized polygalactomannan, a cationized hydroxyalkyl cellulose, a diallyl quaternary ammonium salt polymer, a copolymer containing a methacrylamidepropyltrimethylammonium chloride, and a crosslinked cationic polymer, more preferably one or more selected from the group consisting of a cationized hydroxyalkyl cellulose and a crosslinked cationic polymer, further preferably a cationized hydroxyalkyl cellulose, and furthermore preferably a cationized hydroxyethyl cellulose.

<20> The method for treating a keratinous material according to <19>, in which the cleansing agent composition (A) has a content of the cationic polymer of preferably 0.01 to 10% by mass, more preferably 0.05 to 3% by mass, and further preferably 0.1 to 1% by mass.

<21> The method for treating a keratinous material according to any one of <1> to <20>, in which the cleansing agent composition (A) further contains an electrolyte, preferably an inorganic salt, more preferably one or more selected from the group consisting of sodium chloride, potassium chloride, and magnesium chloride, further preferably one or more selected from the group consisting of sodium chloride and potassium chloride, and furthermore preferably sodium chloride.

<22> The method for treating a keratinous material according to <21>, in which the cleansing agent composition (A) has a content of the electrolyte of preferably 0.1 to 10% by mass, more preferably 0.1 to 5% by mass, further preferably 0.2 to 3% by mass, and furthermore preferably 0.3 to 1% by mass.

<23> The method for treating a keratinous material according to any one of <1> to <22>, in which the cleansing agent composition (A) contains an aqueous medium, preferably one or more selected from the group consisting of water, a lower alcohol, and a low molecular diol and triol having 6 or less carbon atoms, and more preferably water.

<24> The method for treating a keratinous material according to <23>, in which the cleansing agent composition (A) has a content of the aqueous medium of preferably 30% by mass or more, more preferably 50% by mass or more, further preferably 70% by mass or more, furthermore preferably 80% by mass or more, and preferably 99.4% by mass or less.

<25> The method for treating a keratinous material according to any one of <1> to <24>, in which in Step (I), when the keratinous material is hair, the cleansing agent composition (A) is applied in an amount at a bath ratio of the cleansing agent composition (A) to the dry mass of the hair to be treated (mass of cleansing agent composition (A) / dry mass of hair to be treated) of preferably 0.005 to 20, more preferably 0.01 to 15, and further preferably 0.05 to 12.

<26> The method for treating a keratinous material according to any one of <1> to <25>, in which after the cleansing agent composition (A) is applied to the keratinous material in Step (I), a step of washing away the cleansing agent composition (A) with water or hot water is performed.

<27> The method for treating a keratinous material according to any one of <1> to <27>, in which the cationic surfactant (b) is one or more selected from the group consisting of (i) an alkyltrimethylammonium salt, (ii) an alkoxyalkyltrimethylammonium salt, (iii) a dialkyldimethylammonium salt, (iv) an alkylamidealkyltrimethylammonium salt, (v) an alkyldimethylamine and a salt thereof, (vi) an alkoxyalkyldimethylamine and a salt thereof, and (vii) an alkylamidealkyldimethylamine and a salt thereof, preferably one or more selected from the group consisting of (i) an alkyltrimethylammonium salt and (vi) an alkoxyalkyldimethylamine and a salt thereof, and more preferably one or more selected from the group consisting of cetyltrimethylammonium chloride (cetrimonium chloride), stearyltrimethylammonium chloride (sterartrimonium chloride), behenyltrimethylammonium chloride, and N,N-dimethyl-3-octadecyloxypropylamine and a salt thereof.

<28> The method for treating a keratinous material according to any one of <1> to <27>, in which the conditioning agent composition (B) has a content of the cationic surfactant (b) of preferably 0.1 to 25% by mass, more preferably 0.5 to 20% by mass, further preferably 1.0 to 20% by mass, furthermore preferably 2.0 to 15% by mass, furthermore preferably 2.0 to 10% by mass, and furthermore preferably 3.0 to 10% by mass.

<29> The method for treating a keratinous material according to any one of <1> to <28>, in which the conditioning agent composition (B) contains a higher alcohol, preferably a higher alcohol having 12 or more and 22 or less carbon atoms, more preferably one or more selected from the group consisting of cetyl alcohol, oleyl alcohol, stearyl alcohol, isostearyl alcohol, 2-octyldodeanol, myristyl alcohol, behenyl alcohol, and cetostearyl alcohol.

<30> The method for treating a keratinous material according to <29>, in which the conditioning agent composition (B) has a content of the higher alcohol of preferably 1.0 to 15% by mass, more preferably 2.0 to 12% by mass, and further preferably 3.0 to 10% by mass.

<31> The method for treating a keratinous material according to any one of <1> to <30>, in which the conditioning agent composition (B) contains an aqueous medium, preferably one or more selected from the group consisting of water, a lower alcohol, a low molecular diol and triol having 6 or less carbon atoms, and more preferably water.

<32> The method for treating a keratinous material according to <31>, in which the conditioning agent composition (B) has a content of the aqueous medium of preferably 30% by mass or more, more preferably 50% by mass or more, further preferably 70% by mass or more, furthermore preferably 75% by mass or more, and preferably 99.9% by mass or less, more preferably 95% by mass or less.

<33> The method for treating a keratinous material according to any one of <1> to <32>, in which in Step (II), the conditioning agent composition (B) is applied to the keratinous material in a wet state.

<34> The method for treating a keratinous material according to any one of <1> to <33>, in which in Step (II), when the keratinous material is hair, the conditioning agent composition (B) is applied in an amount at a bath ratio of the conditioning agent composition (B) to the dry mass of the hair to be treated (mass of conditioning agent composition (B) /dry mass of hair to be treated) of preferably 0.005 to 20, more preferably 0.01 to 15, and further preferably 0.05 to 12.

<35> The method for treating a keratinous material according to any one of <1> to <34>, in which after the conditioning agent composition (B) is applied to the keratinous material in Step (II), a step of washing away the conditioning agent composition (B) with water or hot water is performed.

<36> The method for treating a keratinous material according to <35>, in which after the conditioning agent composition (B) is applied to the keratinous material in Step (II) and before washing away, a step of leaving the keratinous material as it is in the state with the conditioning agent composition (B) applied thereon.

<37> The method for treating a keratinous material according to any one of <1> to <36>, in which a series of treatments of cleansing by Step (I) and conditioning by Step (II) is repeated twice or more, preferably three times or more, more preferably five times or more.

<38> A cosmetic kit for treating a keratinous material, the kit including a cleansing agent composition (A) that contains an internal olefin sulfonic acid or a salt thereof (a) obtained by sulfonating a starting olefin having an average double bond position of position 3.9 or higher and position 4.5 or lower and a conditioning agent composition (B) containing a cationic surfactant (b).

<39> A composition for treating a keratinous material, being applied to a keratinous material before application of a conditioning agent composition (B) containing a cationic surfactant (b), the composition contains an internal olefin sulfonic acid or a salt thereof (a) obtained by sulfonating a starting olefin having an average double bond position of position 3.9 or higher and position 4.5 or lower.

<40> The composition for treating a keratinous material according to <39>, in which the composition for treating a keratinous material containing the component (a) is a cleansing agent composition, a rinse agent composition, a conditioning agent composition, or a treatment agent composition, for a keratinous material.

Examples

The present invention will be described below with reference to examples, but the present invention is not limited to the scope of the examples. Various physical properties in the examples were measured by the following methods.

(i) Method for measuring positions of double bonds in starting olefin

The double bond positions of a starting olefin were measured by gas chromatography (hereinafter abbreviated as GC). Specifically, dimethyl disulfide was allowed to react with the starting olefin to produce a dithio-derivative, and then the components were separated by GC. Then, the positions of double bonds of the starting olefin were determined based on the peak areas of the components.

The apparatus and analytical conditions used in the measurement are as follows.

GC Apparatus: "HP6890" manufactured by Hewlett-Packard Company
Column: "Ultra-Alloy-1HT Capillary Column" 30 m $\times$ 250 $\mu$m $\times$ 0.15 $\mu$m, manufactured by Frontier Laboratories, Ltd.
Detector: Hydrogen flame ionization detector (FID)
Injection temperature: 300°C
Detector temperature: 350°C
He Flow rate: 4.6 mL/min.

(ii) Measurement method of content of sodium internal olefin sulfonate corresponding to sulfonate group bonding position

For a sodium internal olefin sulfonate having a sulfonate group bonded thereto, the content of each sodium internal olefin sulfonate corresponding to the sulfonate group bonding position thereof was measured by a high speed liquid chromatography / mass spectrometer (HPLC-MS). Specifically, hydroxy forms having a sulfonate group bonded thereto were separated by high speed liquid chromatography (HPLC) and each component was identified with a mass spectrometer (MS). Then, the content of each component was determined from the HPLC-MS peak area.

The apparatuses and conditions used for the measurement are as follows.

HPLC Apparatus: "LD20ASXR" manufactured by Shimadzu Corporation
Column: "ODS Hypersil (resistered trademark)" 4.6 $\times$ 250 mm, particle size: 3 $\mu$m, manufactured by Thermo Fisher Sientific
Sample preparation: 1 in 1000 dilution in methanol
Eluent A: 10 mM ammonium acetate-added water
Eluent B: 10 mM ammonium acetate-added methacrylonitrile / water = 95/5 (v/v) solution
Gradient: 0 minute (A/B = 60/40) $\rightarrow$ 15.1 to 20 minutes (30/70) $\rightarrow$ 20.1 to 30 minutes (60/40) MS Apparatus: "LCMS-2020" Shimadzu Corporation
ESI Detection: anion detection, m/z = 321.10 (component (a) having 16 or 18 carbon atoms)
Column temperature: 40°C
Flow rate: 0.5 mL/min
Injection volume: 5 $\mu$L

(iii) Measurement method of mass ratio of hydroxy form / olefin form

The mass ratio of hydroxy form /olefin form of a sodium internal olefin sulfonate was measured by HPLC-MS. Specifically, the hydroxy form and the olefin form were separated by HPLC, and were identified by subjecting each form to MS. Then, the ratio of the forms was determined from the HPLC-MS peak areas.

Apparatuses and conditions used in the measurement are as follows.

HPLC Apparatus: "Agilent Technology 1100" manufactured by Agilent Technologies Japan, Ltd.
Column: "L-column ODS 4.6 × 150 mm" manufactured by Chemicals Evaluation and Research Institute, Japan
Sample preparation: 1 in 1000 dilution in methanol
Eluent A: 10 mM ammonium acetate-added water
Eluent B: 10 mM ammonium acetate-added methanol
Gradient: 0 minute (A/B = 30/70%) → 10 minutes (30/70%) → 55 minutes (0/100%) → 65 minutes (0/100%) → 66 minutes (30/70%) → 75 minutes (30/70%)
MS Apparatus: "Agilent Technology 1100MS SL (G1946D)" manufactured by Agilent Technologies Japan, Ltd.
MS Detection: anion detection, m/z = 60-1600, UV 240 nm

(iv) Measurement method of content of starting olefin

The content of the unreacted starting olefin in a sodium internal olefin sulfonate was measured by GC. Specifically, ethanol and petroleum ether were added to an aqueous sodium internal olefin sulfonate solution to perform extraction, thereby obtaining the olefin in the petroleum ether phase. Then, the quantity of the starting olefin was determined from the GC peak area.

Apparatuses and analytical conditions used in the measurement are as follows. GC Apparatus: "Agilent Technology 6850" manufactured by Agilent Technologies Japan, Ltd. Column: "Ultra-Alloy-1HT capillary column" 15 m × 250 $\mu$m × 0.15 $\mu$m, manufactured by Frontier Laboratories Ltd.

Detector: hydrogen flame ionization detector (FID)
Injection temperature: 300°C
Detector temperature: 350°C
He Flow rate: 3.8 mL/minute

(v) Measurement method of content of inorganic compounds

The content of an inorganic compound was measured by potentiometric titration or neutralization titration. Specifically, the content of $Na_2SO_4$ was determined by measuring sulfate radicals ($SO_4^{2-}$) by potentiometric titration. The content of NaOH was determined by neutralization titration with dilute hydrochloric acid.

Production Example 1 (production of sodium internal olefin sulfonate 1 having 16 carbon atoms)

[Production of starting olefin 1 having 16 carbon atoms]

Into a flask equipped with a stirrer, 7000 g (28.9 mol) of 1-hexadecanol (product name: KALCOL 6098, manufactured by KAO CORPORATION), 350 g (5% by mass relative to the raw material alcohol) of $\gamma$-alumina (manufactured by STREM Chemicals, Inc) as a solid acid catalyst were put, and were reacted with stirring under nitrogen flowing (7000 mL/min) in the system at 280°C for 8 hours. The alcohol conversion after completion of the reaction was 100%.

The resulting crude alkene olefin was transferred to a flask for dilution, and was diluted at 136 to 160°C / 4.0 mmHg to obtain a starting olefin 1 having 16 carbon atoms with an olefin purity of 100%. The double bond distribution of the resulting starting olefin 1 was as follows. C-position 1: 1.8% by mass, C-position 2: 21.8% by mass, C-position 3: 18.7% by mass, C-position 4: 18.6% by mass, C-position 5: 14.3% by mass, C-position 6: 11.4% by mass, C-position 7: 6.8% by mass, C-position 8: 6.8% by mass (the sum of C-position 7 and C-position 8: 13.6% by mass). The average double bond position was 4.17).

[Sulfonation of starting olefin 1]

The starting olefin 1 was placed in a thin-film sulfonation reactor having a jacket outside thereof, and was subjected to a sulfonation reaction with sulfur trioxide gas under the condition where cooling water of 10°C was allowed to flow in the jacket outside the reactor. The molar ratio $SO_3$ / internal olefin in the sulfonation reaction was set to 1.01. The resulting sulfonated product was mixed with an aqueous alkali solution prepared with sodium hydroxide (alkali agent) of 1.04 times by mole relative to the theoretical acid value, and was neutralized at 30°C for 1 hour by a continuous method. The resulting neutralized product was heated in an autoclave at 170°C for 1 hour to perform hydrolysis, thereby obtaining a sodium internal olefin sulfonate 1 having 16 carbon atoms. The content of the starting olefin contained in the resulting sodium internal olefin sulfonate 1 having 16 carbon atoms was 0.4% by mass, and the content of inorganic compounds was 0.39% by mass.

The property values of the resulting sodium internal olefin sulfonate 1 are shown in Table 1.

Table 1

| Sodium internal olefin sulfonate | | Production Example 1 |
|---|---|---|
| Starting olefin | | 1 |
| Distribution of sulfonate groups (% by mass) | Position 1 | 2.0 |
| | Position 2 | 24.8 |
| | Position 3 | 19.1 |
| | Position 4 | 22.0 |
| | Position 5 to 9 | 32.1 |
| | Total | 100.0 |
| Hydroxy form | | 83.9 |
| Olefin form | | 16.1 |

Examples 1 to 4, Comparative Examples 1 to 4 (hair treatment method and evaluation (1)) (Preparation of cleansing agent composition and conditioning agent composition)

Components were blended according to the composition shown in Table 2, and then, were mixed into a uniform state to prepare each of a cleansing agent composition and a conditioning agent composition. The cleansing agent compositions were all adjusted to pH 4.3 at 25°C. The blended amounts shown in the tables in the Examples are all an active component amount (% by mass) of each component.

(Step (I))

A tress having a 5-g mass in a dry state was wetted with a hot water at 35 to 40°C, and then, 2 g of the cleansing agent composition of each example was put-spread thereon and was foamed for 30 seconds to cleanse the tress. Subsequently, the tress was rinsed with flowing water at 35 to 40°C for 30 seconds to wash away the cleansing agent composition.

(Step (II))

Onto the tress treated in Step (I), 2 g of the conditioning agent composition of each example was put-spread, and was adapted. Subsequently, the tress was rinsed with flowing water at 35 to 40°C for 30 seconds to wash away the conditioning agent composition.

The series of operations of the above Step (I) to Step (II) was repeated five times in total to treat the hair. The treated hair bundle was further cleansed with 2 g of the cleansing agent composition of each example used in Step (I), and meanwhile, the lubricity in rinsing, the softness in rinsing, the hard-to-entangle property in rinsing, the setting property after towel drying, the setting property after drying, the quickness of drying, and the durability of conditioning effect were evaluated by the following procedures. The results are shown in Table 2.

The lubricity in rinsing, the softness in rinsing, the hard-to-entangle property in rinsing, the setting property after towel drying, and the setting property after drying were each evaluated in four grades with an evaluation result of a standard tress described below taken as "score 3".

With 2 g of the cleansing agent composition of Comparative Example 2, the tress was cleansed once in the same manner as described above, and 2 g of a plane conditioner having the following composition was put-spread, and was adapted thereon, and was washed away with flowing water at 40°C for 1 minute. The tress was taken as the standard tress. The tress was further cleansed with 2 g of the cleansing agent composition of Comparative Example 2, and meanwhile, the lubricity in rinsing, the softness in rinsing, the hard-to-entangle property in rinsing, the setting property after towel drying, and the setting property after drying were evaluated by the following procedures.

[Composition of plane conditioner]

|  | (% by mass) |
|---|---|
| Cetyltrimethylammonium chloride (*1) | 5.0 |
| Cetyl alcohol (*2) | 5.0 |
| Lactic acid | 2.0 |
| Water | Balance |
| Total | 100.0 |

(*1) "QUARTAMIN 60W" manufactured by KAO CORPORATION
(*2) "KALCOL 6098" manufactured by KAO CORPORATION

(Lubricity in rinsing)

While allowing water at 35 to 40°C to flow on the treated tress, the lubricity on the tress surface was sensorily evaluated according to the following criteria.

    4: Higer lubricity than standard tress
    3: Equal to standard tress
    2: Slightly poorer lubricity than standard tress
    1: Lubricity is not felt.

(Softness in rinsing)

While allowing water of 35 to 40°C to flow on the treated tress, the softness of the tress was sensorily evaluated according to the following criteria.

    4: Higher softness than standard tress
    3: Equal to standard tress
    2: Slightly poorer softness than standard tress
    1: Softness is not felt.

(Hard-to-entangle property of hair in rinsing)

While allowing water of 35 to 40°C to flow on the treated Tress, the hard-to-entangle property of the hair in rinsing was sensorily evaluated according to the following criteria.

    4: Less entangled than standard tress
    3: Equal to standard tress
    2: Slightly more entangled than standard tress
    1: Significantly more entangled.

(Setting property after towel drying)

The setting property after towel drying of the treated tress was sensorily evaluated according to following criteria.

    4: More easily set than standard tress
    3: Equal to standard tress
    2: Slightly less easily set than standard tress
    1: Hardly set.

(Setting property after drying)

The treated tress was dried with a drier "EH-NA94" (TURBO mode) manufactured by Panasonic Corporation while combing the hair with fingers and a comb for 4 minutes at 5 cm from the dryer. The setting property of the hair after complete drying was sensorily evaluated according to the following criteria.

    4: More easily set than standard tress

3: Equal to standard tress
2: Slightly less easily set than standard tress
1: Hardly set

(Quickness of drying)

The treated tress was dried with a drier "EH-NA94" (TURBO mode) manufactured by Panasonic Corporation while combing the hair with fingers 20-40 times/30 seconds at 5 cm from the dryer. The water content of the hair was recorded every 30 seconds and the time until the water content reached 0.5% or less was measured.

(Durability of conditioning effect)

The treated tress was repeatedly subjected only to Step (I) (putting and spreading of the cleansing agent composition, cleansing, and rinsing) using the cleansing agent composition used in each example. The conditioning effect on the tress was sensorily evaluated at every performing of Step (I), the number of cleansing operations by Step (I) during which the conditioning effect on the treated tress persists was evaluated according to the following criteria.

5: Five Times or more
4: Four Times
3: Three Times
2: Twice
1: Once
0: None

Table 2

| (% by mass) | | Example | | | | Comparative Example | | | |
|---|---|---|---|---|---|---|---|---|---|
| | | 1 | 2 | 3 | 4 | 1 | 2 | 3 | 4 |
| Step (I) cleansing agent composition | (a1) Sodium internal olefin sulfonate 1*1 | 12.5 | 12.5 | 12.5 | 12.5 | - | - | - | - |
| | (a') Alkylsulfuric acid ester salt *2 | - | - | - | - | 12.5 | 12.5 | 12.5 | 12.5 |
| | Sodium chloride | 0.5 | 0.5 | 0.5 | 0.5 | 0 | 0 | 0 | 0 |
| | Lactic acid | Adjust | Adjust | Adjust | Adjust | Adjust | Adjust | Adjust | Adjust |
| | Water | Balance | Balance | Balance | Balance | Balance | Balance | Balance | Balance |
| | Total | 100 | 100 | 100 | 100 | 100 | 100 | 100 | 100 |
| Step (II) conditioning agent composition | (b1) Cetyltrimethylammonium chloride *3 | 5.0 | | | | 5.0 | | | |
| | (b2) Stearyltrimethylammonium chloride *4 | | 5.0 | | | | 5.0 | | |
| | (b3) Behenyltrimethylammoniu m chloride *5 | | | 5.0 | | | | 5.0 | |
| | (b4) N,N-Dimethyl-3-octadecyloxypropylamine *6 | | | | 5.0 | | | | 5.0 |
| | Cetyl alcohol *7 | 5.0 | 5.0 | 5.0 | 5.0 | 5.0 | 5.0 | 5.0 | 5.0 |
| | Lactic acid | 2.0 | 2.0 | 2.0 | 2.0 | 2.0 | 2.0 | 2.0 | 2.0 |
| | Water | Balance | Balance | Balance | Balance | Balance | Balance | Balance | Balance |
| | Total | 100 | 100 | 100 | 100 | 100 | 100 | 100 | 100 |

(continued)

| (% by mass) | | Example | | | | Comparative Example | | | |
|---|---|---|---|---|---|---|---|---|---|
| | | 1 | 2 | 3 | 4 | 1 | 2 | 3 | 4 |
| Evaluation results | Lubricity in rinsing | 3 | 3 | 4 | 3 | 1 | 1 | 1 | 1 |
| | Softness in rinsing | 3 | 3 | 4 | 3 | 1 | 1 | 1 | 1 |
| | Hard-to-entangle property of hair in rinsing | 4 | 4 | 4 | 4 | 1 | 1 | 1 | 1 |
| | Setting property after towel drying | 4 | 4 | 4 | 4 | 1 | 2 | 1 | 2 |
| | Setting property after drying | 3 | 3 | 3 | 3 | 1 | 1 | 1 | 1 |
| | Quickness of drying [sec] | 150 | 150 | 90 | 120 | 210 | 210 | 210 | 210 |
| | Durability of conditioning effect | 5 | 5 | 5 | 5 | 1 | 1 | 1 | 1 |

*1 (a1) Sodium internal olefin sulfonate 1: sodium internal olefin sulfonate obtained in Production Example 1 (number of carbon atoms: 16, average double bond position: 4.17) *2 (a') Alkylsulfuric acid ester salt: ammonium polyoxyethylene (1) alkyl (C10-16) ether sulfate, "EMAL 125A" manufactured by KAO CORPORATION *3 (b1) Cetyltrimethylammonium chloride: "QUARTAMIN 60W" manufactured by KAO CORPORATION *4 (b2) Stearyltrimethylammonium chloride: "QUARTAMIN 86W" manufactured by KAO CORPORATION *5 (b3) Behenyltrimethylammonium chloride: "QUARTAMIN 2285E" manufactured by KAO CORPORATION *6 (b4) N,N-Dimethyl-3-octadecyloxypropylamine: "FAMIN DM E-80" manufactured by KAO CORPORATION *7 Cetyl alcohol: "KALCOL 6098" manufactured by KAO CORPORATION

The above evaluation (1) is comparison between the case where a sodium internal olefin sulfonate was used in the cleansing agent composition of Step (I) and the case where an alkylsulfuric acid ester salt was used therein in place of the sodium internal olefin sulfonate.

As can be seen in Table 2, the methods of Examples 1 to 4 in which a sodium internal olefin sulfonate was used in the cleansing agent composition of Step (I) are superior in the effects of the invention as compared to Comparative Example 1 to 4 in which an alkylsulfuric acid ester salt was used in place of the sodium internal olefin sulfonate in the cleansing agent composition of Step (I).

Examples 5 to 12 (hair treatment method and evaluation (2))

(Preparation of cleansing agent composition and conditioning agent composition)

Components were blended according to the composition shown in Table 3, and then, were mixed into a uniform state to prepare each of a cleansing agent composition and a conditioning agent composition. The cleansing agent composition was adjusted to pH 4.3 at 25°C.

(Step (I))

A tress having a 5-g mass in a dry state was wetted with a hot water at 35 to 40°C, and then, 2 g of the cleansing agent composition of each example was put-spread thereon and was foamed for 30 seconds to cleanse the tress. Subsequently, the tress was rinsed with flowing water at 35 to 40°C for 30 seconds to wash away the cleansing agent composition.

(Step (II))

Onto the tress treated in Step (I), 2 g of the conditioning agent composition of each example was put-spread, and was adapted. Subsequently, the tress was rinsed with flowing water at 35 to 40°C for 30 seconds to wash away the conditioning agent composition.

The series of operations of the above Step (I) to Step (II) was repeated five times in total to treat the hair. The treated hair bundle was further cleansed with 2 g of the cleansing agent composition of each example used in Step (I), and meanwhile, the lubricity in rinsing, the softness in rinsing, the hard-to-entangle property in rinsing, the setting property after towel drying, the setting property after drying, the quickness of drying, and the durability of conditioning effect were evaluated by the same procedure as described above. The results are shown in Table 3.

Note that the lubricity in rinsing, the softness in rinsing, the hard-to-entangle property in rinsing, the setting property after towel drying, and the setting property after drying were each evaluated in 5 grades with an evaluation result of the aforementioned standard tress taken as "score 1" and an evaluation result of the tress used in Example 9 taken as "score 5". A level equal to the evaluation result of the standard tress was evaluated as "score 1", a level equal to the evaluation result of Example 9 was evaluated as "score 5", a level slightly better than that of the standard tress was evaluated as "score 2", a level slightly poorer than that of Example 9 was as "score 4", and the intermediate between score 2 and score 4 was as "score 3".

Table 3

| (% by mass) | | Example | | | | | | | |
| --- | --- | --- | --- | --- | --- | --- | --- | --- | --- |
| | | 5 | 6 | 7 | 8 | 9 | 10 | 11 | 12 |
| Step (I) cleansing agent composition | (a1) Sodium internal olefin sulfonate *1 | 0.1 | 0.1 | 0.5 | 0.5 | 12.5 | 12.5 | 15 | 15 |
| | Sodium chloride | 0.5 | 0.5 | 0.5 | 0.5 | 0.5 | 0.5 | 0.5 | 0.5 |
| | Polyquaternium-10*8 | 0.3 | 0.3 | 0.3 | 0.3 | 0.3 | 0.3 | 0.3 | 0.3 |
| | Water | Balance | Balance | Balance | Balance | Balance | Balance | Balance | Balance |
| | Total | 100 | 100 | 100 | 100 | 100 | 100 | 100 | 100 |
| Step (II) conditioning agent composition | (b1) Cetyltrimethylammonium chloride *3 | 2.5 | 15 | 2.5 | 15 | 2.5 | 15 | 2.5 | 15 |
| | Cetyl alcohol *7 | 5.0 | 5.0 | 5.0 | 5.0 | 5.0 | 5.0 | 5.0 | 5.0 |
| | Lactic acid | 2.0 | 2.0 | 2.0 | 2.0 | 2.0 | 2.0 | 2.0 | 2.0 |
| | Water | Balance | Balance | Balance | Balance | Balance | Balance | Balance | Balance |
| | Total | 100 | 100 | 100 | 100 | 100 | 100 | 100 | 100 |
| Evaluation results | Lubricity in rinsing | 3 | 3 | 3 | 3 | 5 | 5 | 5 | 5 |
| | Softness in rinsing | 2 | 3 | 2 | 3 | 5 | 5 | 4 | 5 |
| | Hard-to-entangle property of hair in rinsing | 2 | 3 | 3 | 3 | 5 | 5 | 4 | 5 |
| | Setting property after towel drying | 2 | 3 | 2 | 3 | 5 | 5 | 5 | 4 |
| | Setting property after drying | 2 | 3 | 2 | 4 | 5 | 5 | 5 | 4 |
| | Quickness of drying [sec] | 210 | 180 | 180 | 150 | 120 | 180 | 150 | 90 |
| | Durability of conditioning effect | 2 | 3 | 2 | 4 | 5 | 5 | 4 | 4 |

*1 (a1) Sodium internal olefin sulfonate 1: sodium internal olefin sulfonate (number of carbon atoms: 16, average double bond position: 4.17) obtained in Production Example 1
*3 (b1) Cetyltrimethylammonium chloride: "QUARTAMIN 60W" manufactured by KAO CORPORATION
*7 Cetyl alcohol: "KALCOL 6098" manufactured by KAO CORPORATION
*8 Polyquaternium-10: O-[2-hydroxy-3-(trimethylammonio)propyl] hydroxyethyl cellulose chloride, "CATICELO L-150" manufactured by KAO CORPORATION

EP 4 413 973 A1

The above evaluation (2) was performed under stricter evaluation criteria than in the evaluation (1) to study a more suitable range of the method of the present invention.

In Examples 5 to 12, the content of the sodium internal olefin sulfonate in the cleansing agent composition used in Step (I) and the content of the cationic surfactant in the conditioning agent composition used in Step (II) were changed. As can be seen from Table 3, the methods of Examples 9 to 12, preferably the methods of Examples 9 and 10, provide a superior effect of the invention.

Industrial Applicability

According to the present invention, it is possible to provide a method for treating a keratinous material that can impart a sufficient conditioning effect to the treated keratinous material and in the cleansed hair, that can enhance the lubricity and softness in rinsing, the hard-to-entangle property in rinsing, the rate of drying, and the setting property of dried hair, and furthermore, that is also superior in the durability of the conditioning effect.

**Claims**

1.  A method for treating a keratinous material, comprising the following Step (I) and Step (II) in this order:

    Step (I): a step of applying a cleansing agent composition (A) that comprises an internal olefin sulfonic acid or a salt thereof (a) obtained by sulfonating a starting olefin having an average double bond position of position 3.9 or higher and position 4.5 or lower to a keratinous material;
    Step (II): a step of applying a conditioning agent composition (B) comprising a cationic surfactant (b) to the keratinous material.

2.  The method for treating a keratinous material according to claim 1, wherein the cleansing agent composition (A) has a content of the internal olefin sulfonic acid or a salt thereof (a) of 0.1% by mass or more and 30% by mass or less.

3.  The method for treating a keratinous material according to claim 1 or 2, wherein the conditioning agent composition (B) has a content of the cationic surfactant (b) of 0.5% by mass or more and 20% by mass or less.

4.  A cosmetic kit for treating a keratinous material, the kit comprising:

    a cleansing agent composition (A) comprising an internal olefin sulfonic acid or a salt thereof (a) obtained by sulfonating a starting olefin having an average double bond position of position 3.9 or higher and position 4.5 or lower; and
    a conditioning agent composition (B) comprising a cationic surfactant (b).

5.  A composition for treating a keratinous material, being to be applied to a keratinous material before application of a conditioning agent composition (B) comprising a cationic surfactant (b), the composition comprises an internal olefin sulfonic acid or a salt thereof (a) obtained by sulfonating a starting olefin having an average double bond position of position 3.9 or higher and position 4.5 or lower.

## INTERNATIONAL SEARCH REPORT

| International application No. |
| --- |
| **PCT/JP2022/036341** |

| A. | CLASSIFICATION OF SUBJECT MATTER |
| --- | --- |

*A61K 8/46*(2006.01)i; *A61Q 5/02*(2006.01)i; *A61Q 5/12*(2006.01)i; *A61Q 19/00*(2006.01)i; *A61Q 19/10*(2006.01)i
FI: A61K8/46; A61Q19/10; A61Q19/00; A61Q5/02; A61Q5/12

According to International Patent Classification (IPC) or to both national classification and IPC

| B. | FIELDS SEARCHED |
| --- | --- |

Minimum documentation searched (classification system followed by classification symbols)

A61K8/46; A61Q5/02; A61Q5/12; A61Q19/00; A61Q19/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2015-25113 A (KAO CORP) 05 February 2015 (2015-02-05) <br> paragraphs [0059], [0062], [0066], [0160], [0372], [0377], [0418], [0439] | 1-5 |
| Y | JP 2014-76983 A (KAO CORP) 01 May 2014 (2014-05-01) <br> paragraphs [0006], [0021], [0120]-[0128] | 1-5 |
| A | JP 1-272564 A (LION CORP) 31 October 1989 (1989-10-31) <br> entire text | 1-5 |
| A | JP 60-32759 A (LION CORP) 19 February 1985 (1985-02-19) <br> entire text | 1-5 |
| A | JP 2015-178466 A (L'OREAL) 08 October 2015 (2015-10-08) <br> entire text | 1-5 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **22 November 2022** | **13 December 2022** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2022/036341**

| Patent document cited in search report | | | Publication date (day/month/year) | Patent family member(s) | | | Publication date (day/month/year) |
|---|---|---|---|---|---|---|---|
| JP | 2015-25113 | A | 05 February 2015 | US | 2016/0122441 | A1 | |
| | | | | paragraphs [0133], [0135], [0139], [0329]-[0331], [0446]-[0457], [0596], [0680] | | | |
| | | | | EP | 3010591 | A1 | |
| JP | 2014-76983 | A | 01 May 2014 | US | 2014/0079658 | A1 | |
| | | | | paragraphs [0013], [0023], [0116]-[0127] | | | |
| JP | 1-272564 | A | 31 October 1989 | (Family: none) | | | |
| JP | 60-32759 | A | 19 February 1985 | (Family: none) | | | |
| JP | 2015-178466 | A | 08 October 2015 | WO | 2015/141863 | A1 | |
| | | | | entire text | | | |
| | | | | US | 2017/0079899 | A1 | |
| | | | | EP | 3145483 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015027975 A
- JP 2015178467 A

- WO 2017098637 A

**Non-patent literature cited in the description**

- *J. Am. Oil Chem. Soc,* 1992, vol. 69, 39